# EUROPÄISCHE PATENTSCHRIFT

(11) **EP 1 857 062 B1**
(45) Veröffentlichungstag und Bekanntmachung des Hinweises auf die Patenterteilung: **31.08.2011**
(21) Anmeldenummer: 07017392.7
(22) Anmeldetag: 13.01.2004
(51) Int. Cl.: A61B 17/58, A61B 17/78

(54) **Verriegelungsnagel, insbesondere für Frakturen der proximalen femur**
Locking nail particulary for fractures of the proximal end of the femur
Clou de verrouillage, en particulier pour les fractures de l'extrémité proximale du fémur

(30) Priorität: 07.02.2003 DE 20301902 U
(43) Veröffentlichungstag der Anmeldung: 21.11.2007
(62) Teilanmeldung aus: 04000484.8
(73) Patentinhaber: Stryker Trauma GmbH, 24232 Schönkirchen/Kiel (DE)
(72) Erfinder: Dorawa, Klaus, 24232 Schönkirchen (DE); Speitling, Andreas, 24149 Kiel (DE); Geert Von Oldenburg, 24226 Heikendorf (DE)
(74) Vertreter: Kopf, Korbinian Paul

(56) Entgegenhaltungen:
- DE-C1- 4 341 677
- GB-A- 2 209 947
- US-B1- 6 224 601

## Beschreibung

Die Erfindung bezieht sich auf einen Verriegelungsnagel, insbesondere für Frakturen des proximalen Femurs nach dem Oberbegriff des Anspruchs 1 und ein Verfahren zu dessen Herstellung.

Es ist bekannt, zur Versorgung von Trochanterfrakturen und Frakturen des Schenkelhalses oder des Femurkopfes einen Verriegelungsnagel vorzusehen, der von proximal in den Femur eingetrieben wird und der in einer Schrägdurchbohrung einen Schenkelhalsstift führt, der über den Schenkelhals des Femurs in den Femurkopf eingeführt wird. Es ist bekannt, den Schenkelhalsstift mit einem Gewinde zu versehen, damit er in den Femurkopf eingeschraubt werden kann (Schenkelhalsschraube); es ist jedoch auch bekannt, den Schenkelhalsstift als Klinge auszuführen. Ferner ist bekannt, im proximalen Teil des Verriegelungsnagels einen Verriegelungsstift vorzusehen, der mit dem Schenkelhalsstift so zusammenwirkt, dass dieser sich zwar axial in der Schrägdurchbohrung bewegen, sich jedoch nicht drehen kann.

Die Druckschrift US-6,224,601 B1 zeigt ein osteosynthetisches Hilfsmittel, welches einen Verriegelungsnagel mit einem schrägen Durchgang aufweist. In diesen Durchgang kann ein Gegenstand eingeführt werden, der beispielsweise im menschlichen Oberschenkelknochen fixiert werden kann.

Die Gewichtskraft eines mit einem derartigen Implantat versorgten Patienten wird im wesentlichen von dem Schenkelhalsstift in den Verriegelungsnagel eingeleitet. Es kommt zu einer zusammengesetzten Spannung im belasteten Nagelquerschnitt, die sich aus Biege- und Zugspannungen zusammensetzt. Bei Überlast ist eine Rissbildung zu befürchten, und zwar an der Stelle, an der die höchste Zugspannung auftritt. Die höchste Spannungskonzentration entsteht an den scharfen seitlichen Kanten auf gegenüberliegenden Seiten des Schenkelhalsstiftes, wenn er sich in der Schrägdurchbohrung befindet. Die sogenannte Zeitfestigkeit des Implantats ist mithin abhängig von dem kritischen Bereich mit der scharfkantigen Geometrie der Kanten. Die grössten Zugspannungen treten naturgemäss am Eintrittsende der Bohrung auf.

Der Erfindung liegt die Aufgabe zugrunde, einen Verriegelungsnagel der genannten Art dahingehend zu verbessern, dass seine Zeitfestigkeit erhöht wird, sowie ein Verfahren zu dessen Herstellung zu bestimmen.

Diese Aufgabe wird durch die Merkmale der Patentansprüche 1 und 7 gelöst.

Vorteilhafte Weiterbildungen der Erfindung sind Gegenstand der abhängigen Ansprüche.

Bei dem erfindungsgemässen Verriegelungsnagel sind die auf gegenüberliegenden Seiten des Stiftes (wenn er sich in der Bohrung befindet) liegenden Kanten am Eintrittsende und/oder Austrittsende der Bohrung abgeflacht oder angefast. Sie gehen gerundet und stetig in die benachbarten proximalen und distalen Kantenabschnitte über. Vorzugsweise sind die durch die abgeflachten Kanten gebildeten Flächenabschnitte im distalen und proximalen Endbereich in Seitenansicht im wesentlichen konkav ausgebildet und im Querschnitt annähernd gerade.

Bezüglich der einzelnen Kantenabschnitte am Eintrittsende der Bohrung werden die zum proximalen und zum distalen Ende des Nagels hin gerichteten Kantenabschnitte von solchen unterschieden, die zu den Seiten des Nagels hin liegen. Die letzteren sind im Hinblick auf die Ausbildung von Flächenabschnitten in erster Linie gemeint. Denn bei den bekannten Nägeln stellen diese Kantenabschnitte für die Belastung die am meisten kritischen Abschnitte dar, weil sie Bereich reduzierten Querschnittes liegen. Bei der Erfindung wird im Bereich dieser Kantenabschnitte durch Anfasung etwas Material fortgenommen in der Weise, dass eine Kerbwirkung und damit die Gefahr der Bildung von Spannungsspitzen in diesem Bereich vermieden wird. Trotz der Materialentfernung wird die Belastbarkeit erhöht. Darüber hinaus wird im wesentlichen die gesamte Auflagefläche für den Stift in der Bohrung, insbesondere des Schenkelhalsstiftes in der Schrägdurchbohrung wie sie durch die Geometrie des Nagels und der Bohrung vorgegeben ist, beibehalten.

Es ist zwar denkbar, ausschliesslich in den angesprochenen Kantenbereichen eine Materialentfernung durch eine Abflachung oder Abschrägung vorzusehen. Es ist jedoch vorzuziehen, wenn nach einer Ausgestaltung der Erfindung am Eintritts- oder Austrittsende der Bohrung zur Bildung der abgeflachten Kantenabschnitte eine Kerbe geformt ist, welche nahezu die gesamte umlaufende Kante zumindest des Eintrittsendes anschrägt. Auch diese kann so geometrisch gestaltet sein, dass die Gleitfläche des Stifts im wesentlich auf ganzer Länge erhalten bleibt.

Vorzugsweise bildet die äussere gerundete umlaufende Kante der Kerbe eine im wesentlichen rechteckige oder quadratische Kontur mit abgerundeten Ecken.

Die relativ schmalen, länglichen Flächenabschnitte an den gegenüberliegenden seitlichen Kantenabschnitten gehen vorzugsweise in geschrägte oder gefaste Flächenabschnitte zur proximalen und distalen Seite des Eintritts- oder Austrittsende hin über, die - seitlich gesehen - einen konkaven Verlauf aufweisen und gerundet in die gerundete äussere Kante übergehen.

Die Erfindung ist insbesondere für Verriegelungsnägel geeignet, die in einer proximalen Schrägbohrung einen Schenkelhalsstift aufnehmen. Hierbei ist auch die erfindungsgemässe Entlastung des Eintrittsendes der Schrägbohrung ausreichend. Sie ist jedoch auch für Bohrungen des Verriegelungsnagels geeignet, die senkrecht zur Nagelachse liegen und distal angeordnet sind.

Beim erfindungsgemässen Nagel wird die Zeitfestigkeit des Implantats erhöht, indem die Aussengeometrie der Kanten der Bohrung an den Enden so verändert werden, dass die durch die Belastung im klinischen Einsatz hervorrufenden Spannungen im Material umgeleitet werden, um besonders kritische Bereiche, wie sie mit scharfkantigen Geometrien entstehen, zu entlasten. Die scharfen Kanten werden entfernt und die Spannung wird über eine grössere Fläche verteilt. Dieser Vorteil wird erzielt, ohne dass der Gleitmechanismus für den Stift beeinträchtigt ist.

Wie schon erläutert, verlaufen die Spannungslinien bei einer Durchbohrung in einem Nagel entlang des schwächsten Querschnitts. Dadurch, dass bei der Erfindung eine Abflachung in den seitlichen Kantenabschnitten des Eintrittsendes der Schrägbohrung erfolgt, werden die Spannungslinien im Bereich höherer Festigkeit um den Querschnitt umgeleitet. Hierbei kann auch vorgesehen werden, die umlaufende Kante am Eintrittsende der Bohrung über ihren gesamten Verlauf durch Materialentfemung anzufasen oder anzuschrägen. Daher sieht die Erfindung vor, dass die umlaufende Kante des Eintrittsbereichs eine umlaufende Fase bzw. Schrägfläche aufweist, deren Form und Verlauf dadurch gebildet ist, dass mit Hilfe eines kreisförmigen Fräswerkzeugs, das um seine Achse gedreht wird, die senkrecht auf der Achse des proximalen Nagelabschnitts steht und dessen Durchmesser grösser ist als der Durchmesser der Bohrung, gegen die Kante des Eintrittsendes bewegt wird, bis die umlaufende Fase erzeugt ist. Auf diese Weise wird ebenfalls eine wirksame Spannungsentlastung im Eintrittsende der Bohrung erzielt, ohne dass es zu einer ungünstigen Materialschwächung kommt oder die Auflagefläche des Schenkelhalsstiftes oder eines anderen Stiftes in der Bohrung verringert wird. Der Fertigungsschritt, der hierfür erforderlich ist, ist auf einfache Weise mit einfachen Mitteln möglich.

Ein erster Aspekt der vorliegenden Erfindung ist ein Verriegelungsnagel, insbesondere für Frakturen des proximalen Femur, bei dem im proximalen Abschnitt des Nagels eine vorzugsweise zur Achse des proximalen Abschnitts verlaufende Bohrung vorgesehen ist zur Aufnahme einer Schraube oder eines Stifts, insbesondere eines Schenkelhalsstiftes, dadurch gekennzeichnet, dass die auf gegenüberliegenden Seiten liegenden seitlichen Kantenabschnitte am Eintrittsende (30) oder auch Austrittsende der Bohrung (18) abgeflacht sind und gerundet und stetig in benachbarte Kantentabschnitte zur proximalen und distalen Seite des Eintritts- und/oder Austrittsendes hin übergehen.

Ein weiterer, zweiter Aspekt der vorliegenden Erfindung ist ein Verriegelungsnagel, der nach dem ersten Aspekt ausgestaltet ist und weiterhin dadurch gekennzeichnet ist, dass die durch die abgeflachten Kantenabschnitte gebildeten Flächenabschnitte (38, 40) in Längsrichtung des Nagelabschnitts im Endbereich im wesentlichen konkav verlaufen.

Ein weiterer dritter Aspekt des erfindungsgemäßen Verriegelungsnagels ist ein Verriegelungsnagel nach dem ersten oder zweiten Aspekt der weiter dadurch gekennzeichnet ist, dass am Eintrittsende (30) und/oder Austrittsende der Bohrung (18) zur Bildung der abgeflachten Kantenabschnitt eine Kerbe geformt ist.

Ein vierter Aspekt des erfindungsgemäßen Verriegelungsnagels ist ein Verriegelungsnagel nach dem dritten Aspekt, wobei der Verriegelungsnagel zusätzlich dadurch gekennzeichnet ist, dass die Kerbe derart geformt ist, dass die Gleitfläche des Stiftes in der Bohrung (18) im wesentlichen auf der ganzen Länge der ursprünglichen Bohrung (18) erhalten bleibt.

Ein fünfter Aspekt des erfindungsgemäßen Verriegelungsnagels ist die Ausgestaltung des Nagels nach dem dritten oder vierten Aspekt und ist weiterhin dadurch gekennzeichnet, dass die umlaufende äussere im Querschnitt gerundete Kante (34) der Kerbe eine annähernd rechteckige oder quadratische Kontur hat.

Weiterhin ist ein Verriegelungsnagel nach einem sechsten Aspekt ausgestaltet, wobei dieser Aspekt eine Ausgestaltung nach einem der Aspekte zwei bis fünf beinhaltet und weiterhin dadurch gekennzeichnet ist, dass die annähernd achsparallel verlaufenden seitlichen Flächenabschnitte (38, 40) im mittleren Bereich - in Längsrichtung des proximalen Nagelabschnitts gesehen - annähernd in einer gemeinsamen Ebene liegen, zu der die Längsachse des proximalen Nagelabschnitts (12) annähernd parallel verläuft.

Ein weiterer siebter Aspekt des erfindungsgemäßen Verriegelungsnagels ist eine Ausgestaltung des Nagels nach einem der Aspekte drei bis sechs, wobei diese Ausgestaltung weiterhin dadurch gekennzeichnet ist, dass die auf gegenüberliegenden Seiten des Stiftes liegenden seitlichen Flächenabschnitte (38, 40) der Kerbe zum proximalen Ende des Verriegelungsnagels hin gerundet in einem Querschnitt konkaven Flächenabschnitt (44) übergehen, dessen radial äusserer Kantenabschnitt den proximalen äusseren Kantenabschnitt der Kerbe bildet.

Ein achter Aspekt des Verriegelungsnagels weist ein Ausgestaltung des Nagels nach einem der Aspekte drei bis sieben auf und ist weiterhin dadurch gekennzeichnet, dass die auf gegenüberliegenden Seiten des Stiftes liegenden seitlichen Flächenabschnitte (38, 40) der Kerbe zum distalen Ende des Nagels hin gerundet in einen sich quer zur Nagelachse erstreckenden konkaven Flächenabschnitt übergehen, der annähernd parallel zur Bohrungsachse nach aussen über eine gerundete Kante in die Aussenkontur des Nagels (10) übergeht.

Im Folgenden werden mit Verweis auf die Figuren bevorzugte Ausführungsbeispiele des erfindungsgemäßen Moduls beschrieben.

In der folgenden Figurenbeschreibung werden für die gleichen oder ähnlichen Elemente die gleichen Bezugsziffern verwendet.
Fig. 1 zeigt die Seitenansicht eines Verriegelungsnagels nach der Erfindung,
Fig. 2 zeigt einen Schnitt durch den Nagel nach Fig. 1 entlang der Linie 2-2,
Fig. 3 zeigt vergrössert die Draufsicht auf einen Teil des Nagels nach Fig. 1,
Fig. 4 zeigt eine ähnliche Draufsicht wie Fig. 3, jedoch aus einer um 90 DEG versetzten Perspektive,
Fig. 5 zeigt die Seitenansicht des Nagelabschnitts nach Fig. 3,
Fig. 6 zeigt eine Draufsicht auf einen proximalen Abschnitt eines Knochennagels ähnlich den proximalen Abschnitten nach den Fign. 3 bis 5 mit einer Schrägdurchbohrung nach dem Stand der Technik,
Fig. 7 zeigt die gleiche Ansicht nach einer Anfasung der umlaufenden Kante des Eintrittsendes der gezeigten Schrägbohrung,
Fig. 8 zeigt eine Seitenansicht des Nagelabschnitts nach Fig. 7.

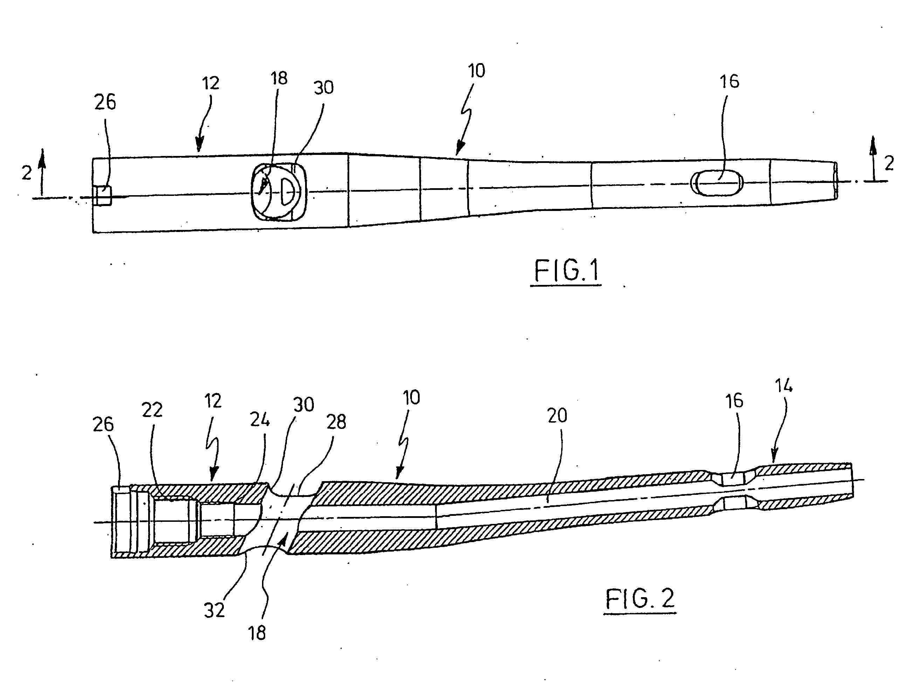

Einer in den Fign. 1 und 2 dargestellter Verriegelungsnagel 10 weist einen proximalen Abschnitt 12 und einen distalen Abschnitt 14 auf. Letzterer enthält eine längliche Querbohrung 16 zur Aufnahme einer nicht dargestellten Verriegelungsschraube. Der proximale Abschnitt weist eine Schrägdurchbohrung 18 auf zur Aufnahme eines nicht gezeigten Schenkelhalsstiftes. Vorstehend und nachstehend ist stets von einem Schenkelhalsstift die Rede, wobei dieser allgemein alle üblichen Schenkelhalsschrauben und -stifte erfassen soll, welche bisher bekannt geworden sind.

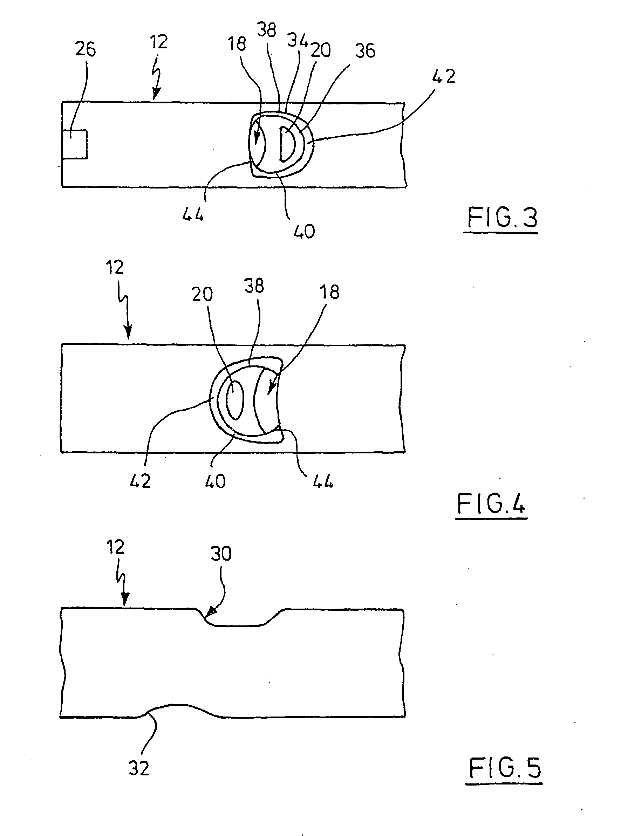

Der Nagel 12 ist komplett mit einer axialen Durchbohrung 20 geformt, und am proximalen Ende sind zwei im Durchmesser unterschiedliche Gewindeabschnitte 22, 24 vorgesehen, wobei der innen liegende zur Aufnahme eines nicht gezeigten Verriegelungsstifts für den Schenkelhalsstift dient und das Gewinde 22 zur Aufnahme eines nicht gezeigten Einschlag- und Zielinstruments zur Anbringung des Nagels 10 über das proximale Femur. Eine radiale Ausnehmung 26 am proximalen Ende dient zur Orientierung des Einschlag- und Zielinstruments am Nagel 10 in Drehrichtung. Die erwähnten konstruktiven Merkmale sind Stand der Technik. Die nachfolgende Beschreibung richtet sich auf die Schrägbohrung 18. Diese hat ein Eintrittsende 30 und ein Austrittsende 32 für den nicht gezeigten Schenkelhalsstift. Das Eintrittsende ist vom Kopf des Femurs abgewandt, wenn der Nagel proximal in den Femur eingetrieben ist. Die nachfolgenden Erörterungen beziehen sich ausschliesslich auf das Eintrittsende, das in Fig. 1 zu erkennen, jedoch in den Fign. 3 und 4 deutlicher herausgestellt ist.

Wie aus den Figuren hervorgeht, ist im Bereich des Eintrittsendes 30 in Draufsicht eine Kerbe eingeformt, deren äussere gerundete Kante 34 annähernd quadratische Kontur hat mit abgerundeten Ecken. Die Kante 34 liegt naturgemäss in der Aussenkontur des proximalen Nagelabschnitts 12 und ausserhalb der Kante, welche durch die Bohrung 18 gebildet wird. Die radial innerhalb der äusseren Kante 34 liegende innere Kante der Kerbe, welche durch die Bohrung 18 gebildet ist, ist mit 36 bezeichnet. Zwischen der äusseren Kante 34 und der inneren Kante 36 sind zum Teil abgeschrägte oder gefaste Flächenabschnitte ausgebildet. Erste seitliche Flächenabschnitte 38, 40 liegen auf gegenüberliegenden Seiten des nicht gezeigten Schenkelhalsstiftes und erstrecken sich in Draufsicht annähernd parallel zur Aussenseite des proximalen Abschnitts 12. Die Flächenabschnitte 38, 40 liegen zumindest im mittleren Bereich annähernd in einer gemeinsamen Ebene, wie sich aus Fig. 2 ergibt, die annähernd parallel zur Längsachse des proximalen Abschnitts 12 verläuft. Nach proximal und distal hin gehen die Flächenabschnitte 38, 40 stetig in Flächenabschnitte 42, 44 über. Der Flächenabschnitt 42 liegt distal und der Flächenabschnitt 44 proximal. Wie aus Fig. 2 zu erkennen, ist der Konturverlauf der Flächenabschnitte 38, 40 im Längsschnitt schwach konkav, insbesondere zu den Enden hin und die Flächenabschnitte 38, 40 gehen stetig in einen Flächenabschnitt über, der proximal durch die Bohrung 18 gebildet ist. Der äussere proximale Kantenabschnitt entspricht im wesentlichen dem Verlauf des Kantenabschnitts, der ohnehin durch die Bohrung 18 gebildet ist.

Die beschriebene Formung oder Auskerbung im Eintrittsbereich für den Schenkelhalsstift in die Schrägbohrung 18 ist fertigungstechnisch relativ einfach herstellbar und sichert eine deutliche Reduzierung der Spannungsspitzen in den Endbereichen der Bohrung 18 bei der Belastung des Schenkelhalsstiftes in der Bohrung 18 durch die Gewichtskraft des Patienten, insbesondere am Eintrittsende, ohne dass die Gleitfläche des Schenkelhalsstiftes, d.h. die Auflagefläche für den Schenkelhalsstift in der Schrägbohrung 18, merklich verringert ist. Die Materialwegnahme am Eintrittsende 30 lässt sich anhand von Fig. 5 leicht erkennen. Die Durchdringungslinie der Schrägbohrung 18 in der Aussenkontur des proximalen Nagelabschnitts 12 ist durch die Materialwegnahme bzw. Anfasung der Kantenbereiche des Eintrittsendes 30 abgewandelt. Man erkennt, dass relativ wenig Material entfernt wurde, so dass die Materialschwächung hierdurch vernachlässigbar ist und durch die Vorteile der günstigen Krafteinleitung vom Schenkelhalsstift in den Nagel weit überwogen wird. Entscheidend für die Reduzierung der Spannungsspitzen sind die Flächenabschnitte 38,40.

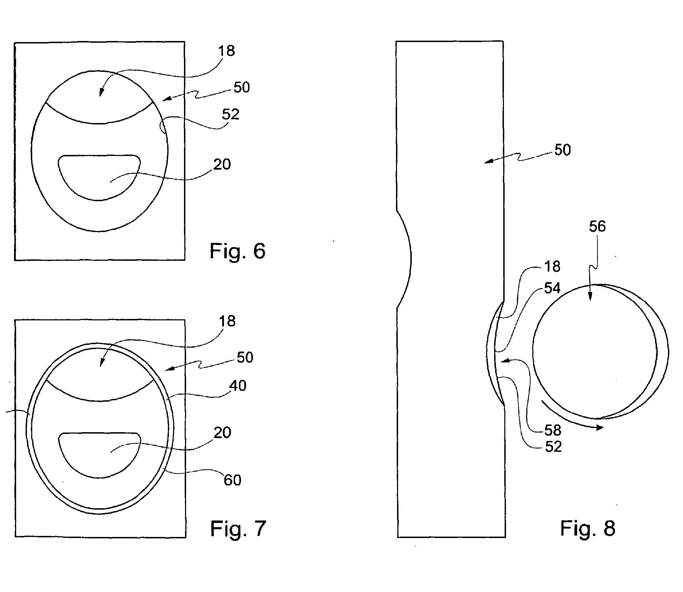

In Fign. 6 bis 8 ist ein proximaler Nagelabschnitt 50 dargestellt, etwa eines Femurnagels gemäss den Fign. 1 und 2. In den Fign. 6 und 7 ist das jeweils obere Ende zum proximalen Ende des Nagels hin gezeigt, während das untere Ende zum distalen Ende hin zeigt. Die Schrägbohrung ist ebenfalls mit 18 angegeben. Auch die axiale Durchbohrung ist mit dem gleichen Bezugszeichen 20 wie in den Fign. 2 bis 4 angegeben. Die Blickrichtung der Seitenansichten nach den Fign. 6 und 7 ist senkrecht zur Nagelachse. Fig. 6 zeigt den Zustand des Nagelabschnitts 50 nach dem Herstellen der Schrägdurchbohrung und der axialen Durchbohrung 20. Fig. 7 zeigt die Verformung der umlaufenden Kante 52 des Eintrittsendes für einen nicht gezeigten Schenkelhalsstift, der in die Schrägbohrung 18 eingeführt wird nach dem Anfasen oder der Abflachung mit einem Fräswerkzeug, wie dies anhand von Fig. 8 noch beschrieben werden soll.

Fig. 8 zeigt die Seitenansicht des Nagelabschnitts 50 annähernd senkrecht zur Schrägdurchbohrung 18, wobei jedoch der Nagelabschnitt 50 um etwa 5 DEG in Uhrzeigerrichtung verdreht worden ist. Dadurch wird am Eintrittsende 54 der Schrägbohrung 18 der Verlauf der Kante 52 deutlicher erkennbar. In Fig. 8 ist ferner ein kreisförmiges Fräswerkzeug 56 zu erkennen, das um eine Achse gedreht wird, die senkrecht auf der Achse des Nagelabschnitts 50 steht. Die axiale Erstreckung des Fräswerkzeugs 56 ist grösser als der Durchmesser der Schrägbohrung 18. Der Durchmesser des kreisförmigen Fräswerkzeugs 56 ist ebenfalls grösser als der Durchmesser der Schrägbohrung 18. Bei der Bearbeitung wird das Fräswerkzeug in Richtung des Pfeils 58 auf die Kante 52 zu bewegt. Das Fräswerkzeug 56 taucht dabei annähernd mittig in das Eintrittsende 54 der Bohrung 18 ein und schrägt die umlaufende Kante 54 umlaufend an, wie bei 60 in Fig. 7 zu erkennen. Insbesondere werden auch hier seitliche gegenüberliegende Flächenabschnitte 38, 40 geformt, welche die Kantenabschnitte der umlaufenden Kante 52 in diesem Bereich anschrägen oder anfasen, um die Spannungsspitzen in diesem Bereich des Nagelabschnitts herabzusetzen.

## Patentansprüche

1. Verriegelungsnagel, insbesondere für Frakturen des proximalen Femurs,
bei dem im proximalen Abschnitt des Nagels eine vorzugsweise schräg zur Achse des proximalen Nagelabschnitts verlaufende Bohrung vorgesehen ist zur Aufnahme einer Schraube oder eines Stiftes, insbesondere eines Schenkelhatsstiftes, **dadurch gekennzeichnet, dass** die Kante eines Eintrittsendes (54) eine umlaufende Fase (60) bzw. Schrägfläche aufweist, dessen Form und Verlauf **dadurch** gebildet ist, dass mit Hilfe eines kreisförmigen Fräswerkzeugs (56), das sich um eine Achse senkrecht zur Achse des proximalen Abschnitts (12) dreht und das einen Aussendurchmesser hat, der grösser ist als der Durchmesser der Bohrung (18) annähernd mittig gegen das Eintrittsende gegen die Kante (52) des Eintrittsendes (54) annähernd senkrecht zur Achse des proximalen Nagelabschnitts (50) bewegt wird, bis die umlaufende Fase (60) erzeugt ist.

2. Verriegelungsnagel nach Anspruch 1,
wobei im wesentlichen die gesamte Auflagefläche für den Stift in der Bohrung, insbesondere des Schenkelhalsstiftes in der Schrägdurchbohrung wie sie durch die Geometrie des Nagels und der Bohrung vorgegeben ist, beibehalten wird.

3. Verriegelungsnagel nach einem der vorherigen Ansprüche 1 bis 2,
wobei der Nagel weiterhin **dadurch gekennzeichnet ist, dass** durch die abgeflachten Kantenabschnitte gebildeten Flächenabschnitte (38, 40) in Längsrichtung des Nagelabschnitts im Endbereich im wesentlichen konkav verlaufen.

4. Verriegelungsnagel nach einem der vorherigen Ansprüche 1 bis 3,
weiterhin **dadurch gekennzeichnet, dass** die annähernd achsparallel verlaufenden seitlichen Flächenabschnitte (38, 40) im mittleren Bereich - in Längsrichtung des proximalen Nagelabschnitts gesehen - annähernd in einer gemeinsamen Ebene liegen, zu der die Längsachse des proximalen Nagelabschnitts (12) annähernd parallel verläuft.

5. Verriegelungsnagel nach einem der vorherigen Ansprüche 1 bis 4,
wobei die Flächenabschnitte (38, 40), welche die Kantenabschnitte der umlaufenden Kante (52) anschrägen oder anfasen, geformt sind, um die Spannungsspitzen in diesem Bereich des Nagelabschnitts herabzusetzen.

6. Verriegelungsnagel nach einem der vorherigen Ansprüche 1 bis 5,
wobei Kantenabschnitte (30) am Eintrittsende der Bohrung abgeflacht sind und gerundet und stetig in benachbarte Kantenabschnitte zur proximalen und distalen Seite des Eintrittsendes hin übergehen.

7. Verfahren zur Herstellung eines Verriegelungsnagels,
insbesondere für Frakturen des proximalen Femurs, bei dem im proximalen Abschnitt des Nagels eine vorzugsweise schräg zur Achse des proximalen Nagelabschnitts (12) verlaufende Bohrung (18) vorgesehen ist zur Aufnahme einer Schraube oder eines Stiftes, insbesondere eines Schenkelhalsstiftes;
das Verfahren aufweisend die folgenden Schritte:
Bereitstellen eines kreisförmigen Fräswerkzeuges (56), das sich um eine Achse senkrecht zur Achse des proximalen Abschnitts (12) dreht und das einen Außendurchmesser hat, der größer ist als der Durchmesser der Bohrung (18);
Annähernd mittiges Bewegen des Fräswerkzeuges gegen das Eintrittsende gegen die Kante (52) des Eintrittsendes (54) annähernd senkrecht zur Achse des proximalen Nagelabschnitts (50), bis die Kante eines Eintrittsendes (54) eine umlaufende Fase (60) bzw. Schrägfläche aufweist und die umlaufende Fase (60) damit erzeugt ist.

8. Verfahren nach Anspruch 7,
wobei im wesentlichen die gesamte Auflagefläche für den Stift in der Bohrung, insbesondere des Schenkelhalsstiftes in der Schrägdurchbohrung wie sie durch die Geometrie des Nagels und der Bohrung vorgegeben ist, beibehalten wird.

9. Verfahren nach einem der vorherigen Ansprüche 7 bis 8,
wobei der Nagel weiterhin **dadurch gekennzeichnet ist, dass** durch die abgeflachten Kantenabschnitte gebildeten Flächenabschnitte (38, 40) in Längsrichtung des Nagelabschnitts im Endbereich im wesentlichen konkav verlaufen.

10. Verfahren nach einem der vorherigen Ansprüche 7 bis 9,
weiterhin **dadurch gekennzeichnet, dass** die annähernd achsparallel verlaufenden seitlichen Flächenabschnitte (38, 40) im mittleren Bereich - in Längsrichtung des proximalen Nagelabschnitts gesehen - annähernd in einer gemeinsamen Ebene liegen, zu der die Längsachse des proximalen Nagelabschnitts (12) annähernd parallel verläuft.

11. Verfahren nach einem der vorherigen Ansprüche 7 bis 10,
wobei die Flächenabschnitte (38, 40), welche die Kantenabschnitte der umlaufenden Kante (52) anschrägen oder anfasen, geformt sind, um die Spannungsspitzen in diesem Bereich des Nagelabschnitts herabzusetzen.

12. Verfahren nach einem der vorherigen Ansprüche 7 bis 11,
wobei Kantenabschnitte (30) am Eintrittsende der Bohrung abgeflacht sind und gerundet und stetig in benachbarte Kantenabschnitte zur proximalen und distalen Seite des Eintrittsendes hin übergehen.

## Claims

1. A locking nail, in particular for fractures of the proximal femur, a bore which preferably extends inclined to the axis of the proximal nail portion being provided in the proximal portion of the nail in order to receive a screw or a pin, in particular a femoral neck pin, **characterised in that** the edge of an inlet end (54) comprises a peripheral bevel (60) or bevelled surface, of which the shape and course are formed with the aid of a circular milling tool (56) which rotates about an axis perpendicular to the axis of the proximal portion (12) and has an outer diameter which is greater than the diameter of the bore (18), said circular milling tool being moved approximately centrally against the inlet end against the edge (52) of the inlet end (54) approximately perpendicular to the axis of the proximal nail portion (50) until the peripheral bevel (60) is produced.

2. The locking nail according to claim 1, wherein substantially the entire support face for the pin in the bore, in particular the femoral neck pin, is maintained in the inclined through-bore in the manner in which it is predetermined by the geometry of the nail and of the bore.

3. The locking nail according to any one of claims 1 to 2 above, wherein the nail is further **characterised in that** the surface portions (38, 40) formed by the flattened edge portions extend substantially concavely in the longitudinal direction of the nail portion in the end region.

4. The locking nail according to any one of claims 1 to 3 above, further **characterised in that** the lateral surface portions (38, 40) extending approximately axially parallel in the central region - viewed in the longitudinal direction of the proximal nail portion - lie approximately in a common plane, in relation to which the longitudinal axis of the proximal nail portion (12) extends approximately parallel.

5. The locking nail according to any one of claims 1 to 4 above, wherein the surface portions (38, 40), which chamfer or bevel the edge portions of the peripheral edge (52), are shaped so as to reduce the stress concentrations in this region of the nail portion.

6. The locking nail according to any one of claims 1 to 5 above, wherein edge portions (30) are flattened at the inlet end of the bore and merge into adjacent edge portions toward the proximal and distal ends of the inlet end in a rounded and continuous manner.

7. A method for producing a locking nail, in particular for fractures of the proximal femur, a bore (18) which preferably extends inclined to the axis of the proximal nail portion (12) being provided in the proximal portion of the nail in order to receive a screw or a pin, in particular a femoral neck pin;
said method comprising the following steps:
providing a circular milling tool (56) which rotates about an axis perpendicular to the axis of the proximal portion (12) and has an outer diameter which is greater than the diameter of the bore (18);
moving the circular milling tool approximately centrally against the inlet end against the edge (52) of the inlet end (54) approximately perpendicular to the axis of the proximal nail portion (50) until the edge of an inlet end (54) comprises a peripheral bevel (60) or bevelled surface and the peripheral bevel (60) is thus produced.

8. The method according to claim 7, wherein substantially the entire support face for the pin in the bore, in particular the femoral neck pin, is maintained in the inclined through-bore in the manner in which it is predetermined by the geometry of the nail and of the bore.

9. The method according to either claim 7 or claim 8, wherein the nail is further **characterised in that** surface portions (38, 40) formed by the flattened edge portions extend substantially concavely in the longitudinal direction of the nail portion in the end region.

10. The method according to any one of claims 7 to 9 above, further **characterised in that** the lateral surface portions (38, 40) extending approximately axially parallel in the central region - viewed in the longitudinal direction of the proximal nail portion - lie approximately in a common plane, in relation to which the longitudinal axis of the proximal nail portion (12) extends approximately parallel.

11. The method according to any one of claims 7 to 10 above, wherein the surface portions (38, 40), which chamfer or bevel the edge portions of the peripheral edge (52), are shaped so as to reduce the stress concentrations in this region of the nail portion.

12. The method according to any one of claims 7 to 11 above, wherein edge portions (30) are flattened at the inlet end of the bore and merge into adjacent edge portions toward the proximal and distal ends of the inlet end in a rounded and continuous manner.

## Revendications

1. Clou de verrouillage, en particulier pour des fractures du fémur proximal, dans lequel il est prévu, dans la section proximale du clou, un alésage s'étendant de préférence en oblique par rapport à l'axe de la section de clou proximale, pour recevoir une vis ou une broche, en particulier une broche pour col fémoral, **caractérisé en ce que** l'arête d'une extrémité d'entrée (54) présente un chanfrein périphérique (60) et/ou biseau, dont la forme et l'allure sont formées à l'aide d'un outil de fraisage circulaire (56), qui tourne autour d'un axe perpendiculairement à l'axe de la section proximale (12) et dont le diamètre extérieur est plus grand que le diamètre de l'alésage (18), est déplacé sensiblement au centre par rapport à l'extrémité d'entrée en direction de l'arête (52) de l'extrémité d'entrée (54), sensiblement perpendiculairement à l'axe de la section de clou proximale (50), jusqu'à ce que le chanfrein périphérique (60) soit produit.

2. Clou de verrouillage suivant la revendication 1, dans lequel la totalité de la surface d'appui pour la broche dans l'alésage, en particulier de la broche pour col fémoral, telle qu'elle est prédéfinie par la géométrie du clou et de l'alésage, est essentiellement maintenue dans le perçage oblique.

3. Clou de verrouillage suivant l'une des revendications précédentes 1 à 2, dans lequel le clou se **caractérise en outre en ce que** des sections superficielles (38, 40), formées par les sections d'arêtes aplaties, sont essentiellement concaves dans la zone d'extrémité, dans la direction longitudinale de la section de clou.

4. Clou de verrouillage suivant l'une des revendications précédentes 1 à 3, **caractérisé en outre en ce que** les sections superficielles latérales (38, 40), s'étendant de façon sensiblement parallèle à l'axe, se situent dans la zone centrale - vue dans la direction longitudinale de la section de clou proximale - approximativement dans un plan commun, par rapport auquel s'étend sensiblement parallèlement l'axe longitudinal de la section de clou proximale (12).

5. Clou de verrouillage suivant l'une des revendications précédentes 1 à 4, dans lequel les sections superficielles (38, 40), qui chanfreinent ou biseautent les sections de l'arête périphérique (52), sont formées pour abaisser les pics de contrainte dans cette zone de la section de clou.

6. Clou de verrouillage suivant l'une des revendications précédentes 1 à 5, dans lequel des sections d'arêtes (30) sont aplaties et arrondies à l'extrémité d'entrée de l'alésage, et se prolongent en continu par des sections d'arêtes voisines en direction du côté proximal et distal de l'extrémité d'entrée.

7. Procédé de fabrication d'un clou de verrouillage, en particulier pour des fractures du fémur proximal, dans lequel il est prévu, dans la section proximale du clou, un alésage (18) s'étendant de préférence en oblique par rapport à l'axe de la section de clou proximale (12), pour recevoir une vis ou une broche, en particulier une broche pour col fémoral;
le procédé comprenant les étapes suivantes :
préparation d'un outil de fraisage circulaire (56), qui tourne autour d'un axe perpendiculairement à l'axe de la section proximale (12) et dont le diamètre extérieur est plus grand que le diamètre de l'alésage (18) ;
déplacement sensiblement central de l'outil de fraisage par rapport à l'extrémité d'entrée en direction de l'arête (52) de l'extrémité d'entrée (54), sensiblement perpendiculairement à l'axe de la section de clou proximale (50), jusqu'à ce que l'arête d'une extrémité d'entrée (54) présente un chanfrein périphérique (60) et/ou biseau et que le chanfrein périphérique (60) soit ainsi produit.

8. Procédé suivant la revendication 7, dans lequel la totalité de la surface d'appui pour la broche dans l'alésage, en particulier de la broche pour col fémoral, telle que prédéfinie par la géométrie du clou et de l'alésage, est essentiellement maintenue dans le perçage oblique.

9. Procédé suivant l'une des revendications précédentes 7 à 8, dans lequel le clou se **caractérise en outre en ce que** des sections superficielles (38, 40), formées par les sections d'arêtes aplaties, sont essentiellement concaves dans la zone d'extrémité, dans la direction longitudinale de la section de clou.

10. Procédé suivant l'une des revendications précédentes 7 à 9, **caractérisé en outre en ce que** les sections superficielles latérales (38, 40), s'étendant de façon sensiblement parallèle à l'axe, se situent dans la zone centrale - vue dans la direction longitudinale de la section de clou proximale - approximativement dans un plan commun, par rapport auquel s'étend sensiblement parallèlement l'axe longitudinal de la section de clou proximale (12).

11. Procédé suivant l'une des revendications précédentes 7 à 10, dans lequel les sections superficielles (38, 40), qui chanfreinent ou biseautent les sections de l'arête périphérique (52), sont formées pour abaisser les pics de contrainte dans cette zone de la section de clou.

12. Procédé suivant l'une des revendications précédentes 7 à 11, dans lequel des sections d'arêtes (30) sont aplaties et arrondies à l'extrémité d'entrée de l'alésage et se prolongent
en continu par des sections d'arêtes voisines en direction du côté proximal et distal de l'extrémité d'entrée.
